# EUROPÄISCHE PATENTSCHRIFT

(11) **EP 2 185 641 B1**
(45) Veröffentlichungstag und Bekanntmachung des Hinweises auf die Patenterteilung: **29.12.2010**
(21) Anmeldenummer: 08802956.6
(22) Anmeldetag: 01.08.2008
(51) Int. Cl.: C08K 5/00, C08K 5/3492, C08K 5/3435, C08K 5/134

(54) **STABILISATORMISCHUNG**
STABILIZER MIXTURE
MÉLANGE STABILISANT

(30) Priorität: 28.08.2007 EP 07115133
(43) Veröffentlichungstag der Anmeldung: 19.05.2010
(73) Patentinhaber: BASF SE, 67056 Ludwigshafen (DE)
(72) Erfinder: SCHAMBONY, Simon, 67061 Ludwigshafen (DE); YAMAZAKI, Hideo, Yokohama Kanagawa 224-0001 (JP); COUGHLIN, Greg, Nanuet, NY 10954 (US)
(86) Internationale Anmeldenummer: PCT/EP2008/060173
(87) Internationale Veröffentlichungsnummer: WO 2009/027180

(56) Entgegenhaltungen:
- EP-A- 0 449 685
- WO-A-2007/082842
- DE-A1- 10 123 732
- DE-A1- 19 859 096
- DE-A1- 19 920 590
- GLASER, ALBAN; SCHAMBONY, SIMON: "Light stabilisation and more. HALS light stabilizers" KUNSTSTOFFE, Bd. 95, Nr. 9, 2005, Seiten 186-190, XP009109064 Carl Hanser Verlag (DE) ISSN: 0023-5563

## Beschreibung

Die Erfindung betrifft Mischungen, die
(a) eine oligomere Verbindung, enthaltend Wiederholeinheiten der allgemeinen Formel (I) oder deren Säureadditionssalze, 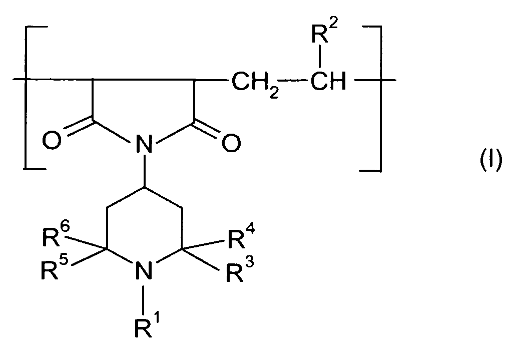 worin,
   - R¹: H, C₁-C₃₀-Alkyl, C₂-C₂₂-Alkenyl, C₁-C₂₀-Alkoxy, Cyanomethyl, 2-Hydroxyethyl, Formyl, C₂-C₆-Alkanoyl, Benzyl oder einen Rest der Formel -CR⁷=CH-CO-OR⁸,
   - R²: H, C₁-C₃₀-Alkyl, Mischung aus C₁₄- bis C₂₈-Alkylgruppen,
   - R³, R⁴, R⁵, R⁶: unabhängig voneinander, gleich oder verschieden, C₁-C₃₀-Alkyl,
   - R⁷: H, C₁-C₆-Alkyl, oder einen Rest der Formel CO-OR⁸,
   - R⁸: C₁-C₁₈-Alkyl, C₃-C₁₅-Cycloakyl, C₇-C₁₈-Aralkyl, Phenyl oder Tolyl,
   bedeuten,
   und/oder
(b) eine Verbindung der allgemeinen Formel (II) oder deren Säureadditionssalze, 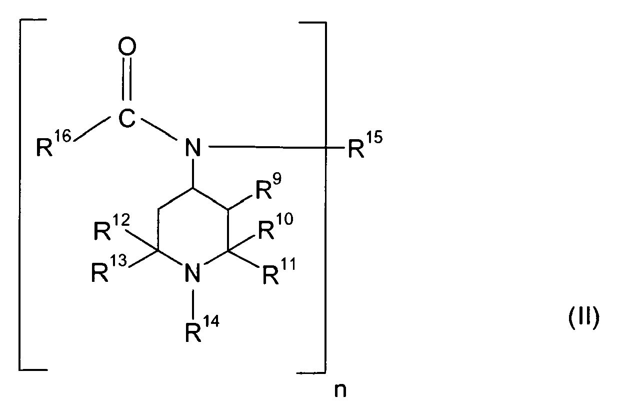 worin,
   - n: 1 oder 2,
   - R⁹: H, C₁-C₄-Alkyl,
   - R¹⁰, R¹¹, R¹², R¹³: unabhängig voneinander, gleich oder verschieden, C₁-C₄-Alkyl oder R¹⁰ und R¹¹ oder R¹² und R¹³ zusammen eine Tetramethylen- oder Pentamethylengruppe,
   - R¹⁴: H, C₁-C₃₀-Alkyl, C₂- C₂₂-Alkenyl, gegebenenfalls durch C₁-C₄-Alkyl, Halogen, C₁-C₄-Alkoxy, Methylendioxy, Ethy- lendioxy und/oder Di-C₁-C₄-alkylamino substituiertes C₇- C₁₂-Phenylalkyl, C₁-C₂₂-Alkanoyl, C₂-C₃-Cyanalkyl, C₁- C₂₂-Hydroxyalkyl oder C₂-C₂₂-Aminoalkyl
   - R¹⁶: H, C₁-C₃₀-Alkyl,
   und
   - wenn n = 1 ist -
   - R¹⁵: H, C₁-C₂₂-Alkyl, C₂-C₂₂-Alkenyl, C₃-C₁₂-Cycloalkyl oder Bicycloalkyl, durch Cyan, Hydroxy oder Carbo-C₁-C₄-alkoxy substituiertes C₂-C₂₂-Alkyl, durch Ethersauerstoff, Stickstoff oder Schwefel unterbrochenes C₄-C₂₂-Alkyl, gegebenenfalls durch C₁-C₄-Alkyl, Halogen, C₁-C₄-Alkoxy, Methylendioxy, Ethy- lendioxy oder Di-C₁-C₄-alkylamino substituiertes C₇-C₂₂-Phenyl- oder Diphenylalkyl, gegebenenfalls durch C₁-C₄-Alkyl oder Carbo-C₁-C₄-alkoxy substituiertes Phe- nyl, ein Rest der allgemeinen Formel (IV) 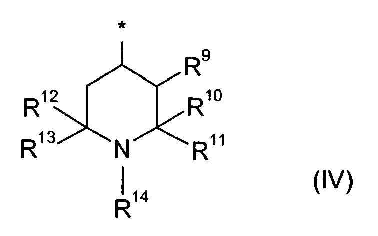 oder heterocyclische Reste enthaltendes C₁-C₂₂-Alkyl,
   oder
   - wenn n = 2 ist -
   - R¹⁵: C₂-C₂₂-Alkylen, C₅-C₂₂-Cycloalkylen, C₈-C₁₄-Phenylalkylen, Phenylen oder durch Ethersauerstoff, Stickstoff, Schwefel oder 5 - oder 6-gliedrige Heterocyclen unterbrochenes C₄-C₃₀-Alkylen
   bedeuten,
   und
(c) wenigstens eine Verbindung der allgemeinen Formel (III), 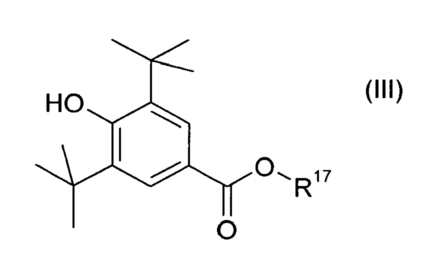 worin,
   - R¹⁷: C₁-C₂₀-Alkyl, Aryl,
   bedeutet,
   und
   optional weitere Zusatzstoffe
enthalten. Weiterhin beschreibt die vorliegende Erfindung Verfahren zur Stabilisierung von unbelebten organischen Materialien, insbesondere Kunststoffen, gegen die Einwirkung von Licht, Sauerstoff und/oder Wärme unter Verwendung dieser Mischung. Des weiteren betrifft die Erfindung Gegenstände, die aus solchermaßen stabilisierten unbelebten organischen Materialien hergestellt werden.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt beziehungsweise ganz bevorzugt sind insbesondere auch diejenigen Ausführungsformen der vorliegenden Erfindung, in denen alle Merkmale des erfindungsgemäßen Gegenstandes die bevorzugten beziehungsweise ganz bevorzugten Bedeutungen haben.

Unbelebtes organisches Materialien, insbesondere Kunststoffe, werden bekanntermaßen sehr schnell, vor allem durch Einwirkung von Licht, Sauerstoff und/oder Wärme zerstört. Diese Zerstörung zeigt sich üblicherweise in Vergilbung, Verfärbung, Rissbildung oder Versprödung des Materials. Mit Lichtschutzmitteln und Stabilisatoren soll daher ein zufriedenstellender Schutz gegen die Zerstörung von unbelebtem organischem Material durch Licht, Sauerstoff und/oder Wärme erzielt werden.

Derivate des 2,2,6,6-Tetraalkylpiperidins sind unter der Bezeichnung HALS (Hindered Amine Light Stabilizers) schon seit ungefähr drei Jahrzehnten als Lichtschutzmittel und Stabilisatoren, insbesondere für Kunststoffe und Lacke, kommerziell im Einsatz.

Aus der WO 2004/046234 A2 sind Stabilisatormischungen, die einen UV-Absorber und mindestens eine weitere Komponente, ausgewählt aus einer Gruppe von fünf Verbindungsklassen enthalten, bekannt. Als UV-Absorber sind unter anderem Benzoate genannt und eine der weiteren Komponenten können auch sterisch gehinderte Amine sein. Eine Kombination aus Hydroxy-Benzoaten mit HALS-Verbindungen ist nicht offenbart.

JP 2003253083 beschreibt ein Propylen-Ethylen Block-Copolymer, das 0,03-3% einer Mischung aus Alkylbenzoat und HALS enthält.

Aus der JP 10195258 sind Agrarfolien aus Polyolefinen, die mit HALS und Benzoaten stabilisiert sind, bekannt.

Das US Patent 6,897,250 B1 beschreibt Automobilteile aus themoplastischen Elastomeren, die 0,001-10% Alkylbenoate und 0,1-0,5% HALS enthalten.

Obwohl sich diese Verbindungen und Mischungen in der kommerziellen Praxis bereits hervorragend bewähren, bleibt doch Raum für Verbesserungen, insbesondere was die Stabilität gegenüber Licht mit einem hohen UV-Anteil betrifft.

Eine Aufgabe der vorliegenden Erfindung war es daher Mischungen bereitzustellen, die eine verbesserte Stabilisierung von unbelebter organischer Materie gegenüber Licht mit einem hohen UV-Anteil und/oder hoher Lichtintensität gewährleisten. Eine weitere Aufgabe der Erfindung war es, Mischungen bereitzustellen die ein hohes Maß an Stabilisierung bieten und auf einfach zugänglichen Ausgangsstoffen beruhen. Eine weitere Teilaufgabe der vorliegenden Erfindung war es Mischungen bereitzustellen, die eine verbesserte Stabilität von unbelebter organischer Materie gegenüber Sauerstoff oder Wärme gewährleisten.

Dementsprechend wurden die eingangs beschriebenen Mischungen gefunden.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Halogen steht für Fluor, Chlor, Brom, oder Iod, vorzugsweise für Fluor, Chlor, oder Brom, besonders bevorzugt für Fluor oder Chlor.

Im einzelnen haben die verschiedenen angegebenen Sammelbegriffe folgende Bedeutung:
C₁-C₃₀-Alkyl: geradkettige oder verzweigte Kohlenwasserstoffreste mit bis zu 30 Kohlenstoffatomen, beispielsweise C₁-C₁₈-Alkyl, C₁-C₁₀-Alkyl oder C₁₁-C₂₀-Alkyl, bevorzugt C₁-C₁₀-Alkyl beispielsweise C₁-C₆-Alkyl, C₁-C₄-Alkyl, C₁-C₃-Alkyl, wie Methyl, Ethyl, n-Propyl, Isopropyl, oder C₄-C₆-Alkyl, n-Butyl, sec-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Tri-methylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethylbutyl, Nonyl oder Decyl (z.B. 2-Propylheptyl) sowie deren Isomere.
C₂-C₂₂-Alkenyl: ungesättigte, geradkettige oder verzweigte Kohlenwasserstoffreste mit 2 bis 22 Kohlenstoffatomen und mindestens einer Doppelbindung, bevorzugt einer Doppelbindung, in einer beliebigen Position, beispielsweise C₂-C₁₀-Alkenyl oder C₁₁-C₂₂-Alkenyl, bevorzugt C₂-C₁₀-Alkenyl wie C₂-C₄-Alkenyl, wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, oder C₅-C₆-Alkenyl, wie 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-bu-tenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1 ,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl oder 1-Ethyl-2-methyl-2-propenyl, sowie C₇-C₁₀-Alkenyl, wie die Isomere von Heptenyl, Octenyl, Nonenyl oder Decenyl.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl sowie ein gesättigtes oder ungesättigtes cyclisches System wie z. B. Norbornyl oder Norbenyl. Besonders bevorzugt C₅-C₆-Cycloalkyl.

C₁-C₂₂-Alkanoyl: eine geradkettige oder verzweigte Alkylgruppe mit 1 bis 22 Kohlenstoffatomen (wie vorstehend genannt), welche über eine Carbonylgruppe (-CO-) angebunden sind, beispielsweise C₁-C₁₁-Alkanoyl oder C₁₂-C₂₂-Alkanoyl, bevorzugt C₁-C₁₁-Alkanoyl wie C₁-C₆-Alkanoyl, wie Formyl, Acetyl, n- oder iso-Propionyl, n-, iso-, secoder tert.-Butanoyl, n-, iso-, sec- oder tert.-Pentanoyl, Hexanoyl, oder C₉-C₁₂-Alkanoyl wie n- oder iso-Nonanoyl, oder n-Dodecanoyl.

Aryl: ein ein- bis dreikerniges aromatisches Ringsystem enthaltend 6 bis 14 Kohlenstoffringglieder, z. B. Phenyl, Hydroxyphenyl, Naphthyl oder Anthracenyl, bevorzugt ein ein- bis zweikerniges, besonders bevorzugt ein einkerniges aromatisches Ringsystem.

Substituierte Aryle: Aryle, die an beliebiger Position, allerdings nicht mehr als fünfmal, bevorzugt nicht mehr als viermal, besonders bevorzugt nicht mehr als dreimal und ganz besonders bevorzugt zweimal oder einmal durch C₁-C₂₀-Alkyl oder Hydroxy substituiert sein können.

Aralkyl: arylsubstituierte Alkyle. Beispielsweise Naphthylmethyl, Diphenylmethyl oder Methylbenzyl, insbesondere 1-Phenylethyl, 2-Phenylethyl, 1-Phenylpropyl, 2-Phenylpropyl, 3-Phenylpropyl, 2-Phenyl-prop-2-yl, 4-Phenylbutyl, 2,2-Dimethyl-2-phenylethyl, 5-Phenylamyl, 10-Phenyldecyl, 12-Phenyldodecyl oder vor allem Benzyl. Bevorzugt C₇-C₁₈-Aralkyl: arylsubstituierte Alkyle mit 7 bis 18 Kohlenstoffatomen.

Als Tolylreste kommen ortho-, meta und vor allem p-Tolyl in Betracht.

Heterocyclen: fünf- bis zwölfgliedrige, bevorzugt fünf- bis neungliedrige, besonders bevorzugt fünf- bis sechsgliedrige, Sauerstoff-, Stickstoff- und/oder Schwefelatome, gegebenenfalls mehrere Ringe aufweisende Ringsysteme wie Furyl, Thiophenyl, Pyrryl, Pyridyl, Imidazoyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl.

C₁-C₂₀-Alkoxy bedeutet eine geradkettige oder verzweigte Alkylgruppe mit 1 bis 20 Kohlenstoffatomen (wie vorstehend genannt), welche über ein Sauerstoffatom (-O-) angebunden sind, beispielsweise C₁-C₁₀-Alkoxy wie n-Hexoxy, iso-Hexoxy, n-Octoxy, 2-Ethylhexoxy und iso-Octoxy, daneben aber auch Methoxy, Ethoxy, n-Propoxy, iso-Propoxy, n-Butoxy, iso-Butoxy, sec.-Butoxy, tert.-Butoxy, n-Pentoxy, n-Nonoxy, n-Decoxy, oder C₁₁-C₂₀-Alkoxy wie n-Undecoxy und n-Dodecoxy, bevorzugt C₁-C₁₀-Alkyloxy, insbesondere bevorzugt C₁-C₃-Alkoxy, wie beispielweise Methoxy, Ethoxy, Propoxy.

C₁-C₂₂-Alkylen: geradkettige oder verzweigte Kohlenwasserstoffreste mit 1 bis 22 Kohlenstoffatomen, beispielsweise C₂-C₁₀-Alkylen oder C₁₁-C₂₂-Alkylen, bevorzugt C₂-C₁₀-Alkylen, insbesondere Methylen, Dimethylen, Trimethylen, Tetramethylen, Pentamethylen oder Hexamethylen.

C₅-C₂₂-Cycloalkylen: geradkettige oder verzweigte Kohlenwasserstoffreste mit 5 bis 22 Kohlenstoffatomen, wobei ein Alkylen durch eine Cycloalkylgruppe unterbrochen ist, d.h. zwei Wasserstoffe der Cycloalkylgruppe durch Alkylene ersetzt sind.

C₈-C₁₄-Phenylalkylen: geradkettige oder verzweigte Kohlenwasserstoffreste mit 8 bis 14 Kohlenstoffatomen, wobei die Alkylenkette durch eine Phenylengruppe unterbrochen ist.

C₁-C₂₂-Hydroxyalkyl: ein an beliebiger Position durch Hydroxygruppe substituiertes C₁-C₂₂-Alkyl.

C₁-C₂₂-Aminoalkyl: ein an beliebiger Position durch eine Aminogruppe substituiertes C₁-C₂₂-Alkyl.

Die im einzelnen aufgeführten Substituenten R¹ bis R¹⁷ können jeweils an beliebiger Position durch ein oder mehrere Heteroatome unterbrochen sein, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch C₁-C₃₀-Alkyl, C₁-C₂₀-Alkoxy, Aryl, Heterocyclen, Heteroatome oder Halogen substituiert sein können, wobei diese ebenfalls maximal zweimal, bevorzugt maximal einmal mit den genannten Gruppen oder substituierten Arylen substituiert sein können.

Die in dieser Gruppe genannten Verbindungsklassen C₁-C₃₀-Alkyl, C₁-C₂₀-Alkoxy, Aryl und Heterocyclen haben die zuvor genannte Bedeutung.

Heteroatome sind bevorzugt Sauerstoff, Stickstoff, Schwefel oder Phosphor.

Säureadditionssalze sind die Salze der jeweiligen Verbindungen mit Mineralsäuren, wie beispielsweise HCl, oder organischen Säuren, wie beispielsweise Essigsäure.

In den erfindungsgemäßen Mischungen kann Komponente (a) ein oder mehrere verschiedene oligomere Verbindungen, enthaltend Wiederholeinheiten der allgemeinen Formel (I) oder deren Säureadditionssalze umfassen.

Weiterhin kann in den erfindungsgemäßen Mischungen Komponente (b) ein oder mehrere verschiedene Verbindung der allgemeinen Formel (II) oder deren Säureadditionssalze umfassen.

Weiterhin kann in den erfindungsgemäßen Mischungen Komponente (c) ein oder mehrere verschiedene Verbindung der allgemeinen Formel (III) und Komponente (d) optional einen oder mehrere Zusatzstoffe umfassen.

Die oligomeren Verbindungen der Komponente (a) weisen im Allgemeinen ein mittleres Molekulargewicht von 1000 bis 50000, bevorzugt von 1500 bis 10000, insbesondere von 3000 bis 5000 auf. Bei den Molekulargewichtsangaben handelt es sich um zahlengemittelte mittlere Molekulargewichte.

Die durchschnittliche Zahl der Wiederholeinheiten in den oligomeren Verbindungen beträgt 3 bis 100, bevorzugt 4 bis 30, insbesondere 5 bis 10.

Der Rest R¹ ist bevorzugt H, C₁-C₆-Alkyl, Formyl, Acyl, C₁-C₆-Alkoxy, Benzyl, oder ein Rest der Formel -CH=CH-CO-R', wobei R' C₁-C₆-Alkyl, insbesondere Methyl oder Ethyl, bedeutet. Besonders bevorzugt ist R¹ H, Methyl, C₁-C₆-Alkoxy, insbesondere H.

Der Rest R² ist bevorzugt C₁₂-C₃₀-Alkyl. Weiterhin kann der Rest R² eine Mischung aus C₁₄-C₂₈-Alkylgruppen sein, vorzugsweise C₁₆-C₂₄-Alkyl, insbesondere C₁₈-C₂₂-Alkyl. Bei R² handelt es sich vorzugsweise um lineare Alkylgruppen.

Das Vorliegen einer Mischung von Alkylgruppen für R² ist so zu verstehen, dass im statistischen Mittel über die Gesamtzahl aller vorliegenden Alkylgruppen zwei bestimmte Alkylgruppen, die sich um nicht mehr als zwei C-Atome unterscheiden dürfen, jeweils mindestens 30 %, vorzugsweise jeweils mindestens 40 % dieser Mischung ausmachen. Insbesondere sind dies Mischungen von 3 bestimmten Alkylgruppen, zum Beispiel Octadecyl, Eicosyl und Docosyl, wobei zwei dieser Gruppen, die sich um 2 C-Atome unterscheiden, mehr als 40 % und die dritte Gruppe 3 bis 18 % der Mischung ausmachen; hierbei können weitere Alkylgruppen mit etwas weniger als 18 oder etwas mehr als 22 C-Atomen in geringfügigen Mengen, üblicherweise weniger als 2 %, in der Mischung vorliegen.

Die Reste R³, R⁴, R⁵ und R⁶ sind bevorzugt C₁-C₄-Alkyl, besonders bevorzugt Methyl, insbesondere bevorzugt haben jeweils alle Reste R³, R⁴, R⁵ und R⁶ die Bedeutung Methyl.

Insbesondere bevorzugt als oligomere Verbindung der Komponente (a) ist eine oligomere Verbindung mit der Wiederholeinheit (la) 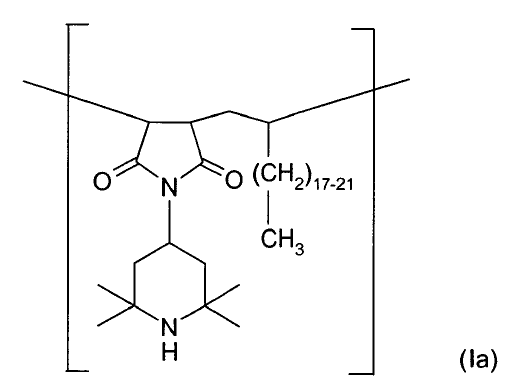 und einer zahlengemittelten Molmasse von 3000 - 4000 g/mol.

Eine solche Verbindung ist unter der Bezeichnung Uvinul® 5050 H von der BASF Aktiengesellschaft, Ludwigshafen, Deutschland erhältlich.

Besonders bevorzugte oligomere Verbindungen der Komponente (a) sind diejenigen, bei denen möglichst viele, insbesondere alle, Substituenten, Symbole und Indizes ihre bevorzugte bzw. besonders bevorzugte Bedeutung annehmen.

Die Herstellung der oligomeren Verbindungen der Komponente (a) kann, falls die Verbindungen nicht kommerziell erhältlich sind, nach den in der WO 94/12544 angegebenen Methoden erfolgen.

Von den Verbindungen der allgemeinen Formel (II) der Komponente (b) sind solche bevorzugt, bei denen die Symbole und Indizes folgende Bedeutungen haben:
R¹⁴ ist bevorzugt H, C₁-C₄-Alkyl, Formyl, Acyl oder Benzyl, besonders bevorzugt H, Methyl, Formyl, Acyl oder Benzyl, insbesondere H.
R¹⁰, R¹¹, R¹² und R¹³ sind bevorzugt C₁-C₄-Alkyl, besonders bevorzugt Methyl, insbesondere bevorzugt sind alle Reste R¹⁰, R¹¹, R¹² und R¹³ Methyl.
R¹⁶ ist bevorzugt H.
n ist bevorzugt 2.

Falls n = 2 ist, so ist R¹⁵ bevorzugt C₂-C₂₂-Alkylen, besonders bevorzugt C₄-C₁₀-Alkylen, ganz besonders bevorzugt C₆-C₈-Alkylen, insbesondere C₆-Alkylen.

Insbesondere bevorzugt als Verbindung der allgemeinen Formel (II) der Komponente (b) ist die Verbindung (IIa), 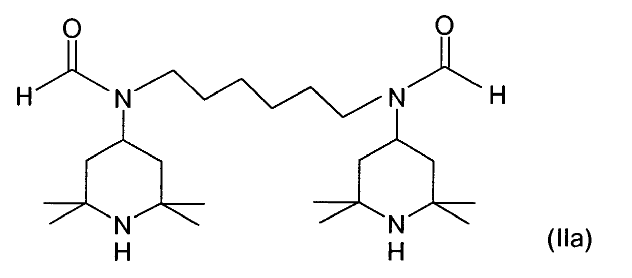 die unter der Bezeichnung Uvinul® 4050 H von der BASF Aktiengesellschaft, Ludwigshafen, Deutschland kommerziell erhältlich ist.

Besonders bevorzugte Verbindungen der allgemeinen Formel (II) der Komponente (b) sind solche, bei denen möglichst viele, insbesondere alle, Substituenten, Symbole und Indizes ihre bevorzugte bzw. besonders bevorzugte Bedeutung annehmen.

Die Herstellung von Verbindungen der allgemeinen Formel (II), insbesondere von Verbindungen der Formel (IIa), kann, soweit sie nicht kommerziell erhältlich sind, nach den in der EP-A 0 316 582 und GB 2311292 beschriebenen Methoden erfolgen.

Von den Verbindungen der allgemeinen Formel (III) der Komponente (c) sind solche bevorzugt, bei denen die Symbole und Indizes folgende Bedeutungen haben:
R¹⁷ ist bevorzugt C₁₀-C₂₀-Alkyl oder ein substituiertes Aryl, ganz bevorzugt C₁₄-C₁₈-Alkyl oder ein substituiertes Phenyl, insbesondere ein C₁₆-Alkyl (C₁₆H₃₃) oder Disubstituiertes-phenyl. Ganz besonders bevorzugt ist R¹⁷ entweder ein lineares C₁₆-Alkyl oder ein 2,4 Di-t-butyl-phenyl.

Die Herstellung von Verbindungen der allgemeinen Formel (III) kann, soweit sie nicht kommerziell erhältlich sind, nach bekannten, dem Fachmann geläufigen Methoden erfolgen, wie beispielsweise in den Schriften US 4128726 bzw. EP 139919 beschrieben.

Das Gewichtsverhältnis der Komponenten (a) und (b) in den erfindungsgemäßen Mischungen beträgt, falls beide Komponenten vorhanden sind, im Allgemeinen von 5:1 bis 1:5, bevorzugt von 2:1 bis 1:2, besonders bevorzugt von 1,2:1 bis 1:1,2, insbesondere bevorzugt ist eine Mischung im Gewichtsverhältnis von etwa 1:1.

Das Gewichtsverhältnis der Komponenten (a) und (c) in den erfindungsgemäßen Mischungen beträgt, falls keine Komponente (b) vorhanden ist, im Allgemeinen von 10:1 bis 1:2, bevorzugt von 5:1 bis 1:1, besonders bevorzugt von 2:1 bis 1:1, insbesondere bevorzugt ist eine Mischung im Gewichtsverhältnis von etwa 2:1.

Das Gewichtsverhältnis der Komponenten (b) und (c) in den erfindungsgemäßen Mischungen beträgt, falls keine Komponente (a) vorhanden ist, im Allgemeinen von 10:1 bis 1:2, bevorzugt von 5:1 bis 1:1, besonders bevorzugt von 2:1 bis 1:1, insbesondere bevorzugt ist eine Mischung im Gewichtsverhältnis von etwa 2:1.

Das Gewichtsverhältnis der Summe der Komponenten (a) und (b) zu Komponente (c) in den erfindungsgemäßen Mischungen beträgt, falls Komponente (a) und Komponente (b) vorhanden sind, im Allgemeinen von 10:1 bis 1:2, bevorzugt von 5:1 bis 1:1, besonders bevorzugt von 2:1 bis 1:1 insbesondere bevorzugt ist eine Mischung im Gewichtsverhältnis von etwa 2:1.

Die Herstellung der erfindungsgemäßen Mischungen kann nach bekannten, dem Fachmann geläufigen Verfahren erfolgen.

Vorzugsweise kann man die Komponente (c) einer Schmelze der Komponenten (a) und/oder (b) zusetzen, homogenisieren, in die gewünschte Form, beispielsweise Pastillen, bringen und erkalten lassen.

Man kann aber auch Lösungen der Komponenten (c) und (a) und/oder (b) mischen und das oder die Lösungsmittel dann entfernen.

Auch ein Vermengen der Produkte, die gegebenenfalls vorher gemahlen wurden, ist möglich. Die Mischung kann gegebenenfalls auch nach dem Mischen in eine geeignete Form gebracht werden, zum Beispiel durch Granulierung.

Gegenstand der Erfindung ist daher auch ein entsprechendes Verfahren zur Herstellung der erfindungsgemäßen Mischungen.

Besonders bevorzugt sind die folgenden Kombinationen von Verbindungen der Komponenten (c) und (a) und/oder (b):
die Kombination der Verbindungen (IIIa) oder (IIIb) mit (la), (IIIa) oder (IIIb) mit (IIa) sowie einer Mischung aus (la) und (IIb) mit (IIIa) oder (IIIb)

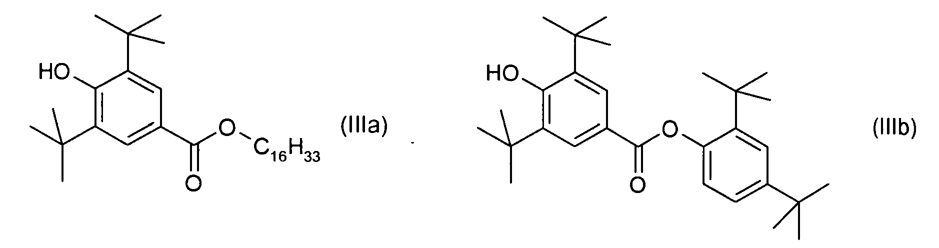

Insbesondere bevorzugt ist die Kombination der Verbindung (la) mit der Verbindung (IIIa) und die Kombination der Verbindungen (la) und (IIIb).

Die erfindungsgemäßen Mischungen eignen sich in hervorragender Weise zur Verwendung als Stabilisatoren zum Stabilisieren von unbelebtem organischem Material gegen die Einwirkung von Licht, Sauerstoff und Wärme. Die erfindungsgemäßen Mischungen werden den zu stabilisierenden unbelebten organischen Materialien in der Regel in einer Konzentration zugesetzt, die ausreicht um die gewünschte Stabilisationswirkung zu erreichen. Bevorzugt werden die erfindungsgemäßen Mischungen in einer Konzentration von 0,01 bis 5 Gew.-%, vorzugsweise von 0,02 bis 1 Gew.-%, bezogen auf das unbelebte organische Material, vor, während oder nach der Herstellung des unbelebten organischen Materials zugesetzt.

Die erfindungsgemäße Mischung kann dem zu schützenden unbelebten organischen Material als vorgefertigte Mischung der Komponenten (a) und/oder (b) und (c) zugesetzt werden, ebenso ist jedoch die getrennte Zugabe der Komponenten (a) und/oder (b) und (c) zu dem zu schützenden Material möglich, die Mischung entsteht dann erst in dem zu schützenden Material. Bei einer getrennten Zugabe der Komponenten (a) und/oder (b) und (c) kann diese gleichzeitig oder zeitlich versetzt erfolgen, wobei die Reihenfolge im Allgemeinen unerheblich ist.

Unter unbelebtem organischem Material sind beispielsweise kosmetische Präparate wie Salben und Lotionen, Arzneimittelformulierungen wie Pillen und Zäpfchen, photografisches Aufzeichnungsmaterial wie photografische Emulsionen oder Vorprodukte für Kunststoffe und Lacke, insbesondere jedoch Kunststoff und Lacke selbst, zu verstehen. Aus unbelebtem organischen Material lassen sich Gegenstände herstellen.

Gegenstand der Erfindung ist außerdem gegen die Einwirkung von Licht, Sauerstoff und Wärme stabilisiertes unbelebtes organisches Material, insbesondere Kunststoffe und Lacke, welches die erfindungsgemäßen Mischungen, vorzugsweise in den oben angegebenen Konzentrationen enthält.

Zur Vermischung der erfindungsgemäßen Mischung vor allem mit Kunststoffen können alle bekannten Vorrichtungen und Methoden zum Einmischen von Stabilisierungsmitteln oder anderen Zusätzen in Polymere angewandt werden.

Wahlweise enthält die erfindungsgemäße Mischung als Komponente (d) oder das durch die Mischung zu stabilisierende unbelebte organische Material zusätzlich wenigstens einen weiteren Lichtstabilisator und/oder weitere (Co)stabilisatoren. Geeignete Lichtstabilisatoren und weitere (Co)stabilisatoren sind beispielsweise aus den Gruppen a) bis s) ausgewählt:
a) 4,4-Diarylbutadiene,
b) Zimtsäureester,
c) Benzotriazole,
d) Hydroxybenzophenone,
e) Diphenylcyanacrylate,
f) Oxamide,
g) 2-Phenyl-1,3,5-triazine,
h) Antioxidantien,
i) Nickelverbindungen,
j) Von den Verbindungen der allgemeinen Formeln (I) und (II) verschiedene sterisch gehinderte Amine,
k) Metalldesaktivatoren,
l) Phosphite und Phosphonite,
m) Hydroxylamine,
n) Nitrone,
o) Aminoxide,
p) Benzofuranone und Indolinone,
q) Thiosynergisten,
r) Peroxid-zerstörende Verbindungen und
s) basische Costabilisatoren.

Zur Gruppe a) der 4,4-Diarylbutadiene zählen beispielsweise Verbindungen der Formel (aa)

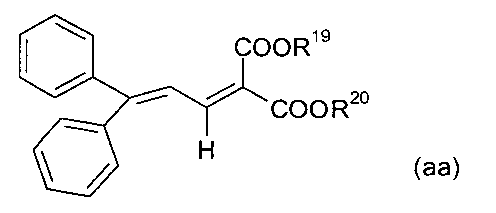

Die Verbindungen sind aus der EP-A-916 335 bekannt. Die Substituenten R¹⁹ und R²⁰ bedeuten, unabhängig voneinander, gleich oder verschieden, bevorzugt C₁-C₈-Alkyl und C₅-C₈-Cycloalkyl.

Zur Gruppe b) der Zimtsäureester zählen beispielsweise 4-Methoxyzimtsäure-2-isoamylester, 4-Methoxyzimtsäure-2-ethylhexylester, Methyl-α-methoxycarbonylcinnamat, Methyl-α-cyano-β-methyl-p-methoxycinnamat, Butyl-α-cyano-β-methyl-p-methoxy-cinnamat und Methyl-α-methoxycarbonyl-p-methoxycinnamat.

Zur Gruppe c) der Benzotriazole zählen beispielsweise 2-(2'-Hydroxyphenyl)-benzotriazole wie 2-(2'-Hydroxy-5'-methylphenyl)-benzotriazol, 2-(3',5'-Di-tert-butyl-2'-hydroxyphenyl)benzotriazol, 2-(5'-tert-Butyl-2'-hydroxyphenyl)-benzotriazol, 2-(2'-Hydroxy-5'-(1,1,3,3-tetramethylbutyl)phenyl)benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octyloxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol und 2-(3'-tert-Butyl-2'-hydroxy-5'-(2-isooctyloxy-carbonylethyl)-phenylbenzotriazol, 2,2'-Methylen-bis[4-(1,1,3,3-tetramethylbutyl)-6-benzotriazol-2-yl-phenol]; das Produkt der Veresterung von 2-[3'-tert-Butyl-5'-(2-methoxycarbonylethyl)-2'-hydroxyphenyl]-2H-benzotriazol mit Polyethylenglycol 300; [R-CH2CH2-COO(CH2)3]2, mit R = 3'-tert-Butyl-4'-hydroxy-5'-2H-benzotriazol-2-yl-phenyl und Gemische davon.

Zur Gruppe d) der Hydroxybenzophenone zählen beispielsweise 2-Hydroxybenzophenone wie 2-Hydroxy-4-methoxybenzophenon, 2,2'-Dihydroxy-4-methoxybenzophenon, 2,4-Dihydroxybenzophenon, 2,2',4,4'-Tetrahydroxybenzophenon, 2,2'-Dihydroxy-4,4'-dimethoxybenzophenon, 2,2'-Dihydroxy-4,4'-dimethoxybenzophenon, 2-Hydroxy-4-(2-ethylhexyloxy)benzophenon, 2-Hydroxy-4-(n-octyloxy)benzophenon, 2-Hydroxy-4-methoxy-4'-methylbenzophenon, 2-Hydroxy-3-carboxybenzophenon, 2-Hydroxy-4-methoxybenzophenon-5-sulfonsäure und deren Natriumsalz, 2,2'-Dihydroxy-4,4'-dimethoxybenzophenon-5,5'-bissulfonsäure und deren Natriumsalz.

Zur Gruppe e) der Diphenylcyanacrylate zählen beispielsweise Ethyl-2-cyan-3,3-diphenylacrylat, das beispielsweise im Handel unter dem Namen Uvinul® 3035 der Fa. BASF AG, Ludwigshafen erhältlich ist, 2-Ethylhexyl-2-cyan-3,3-diphenylacrylat, das beispielsweise im Handel als Uvinul® 3039 der Fa. BASF AG, Ludwigshafen, erhältlich ist und 1,3-Bis-[(2'-cyano-3',3'-diphenylacryloyl)oxy]-2,2-bis{[2'-cyano-3',3'-diphenyl-acryloyl)oxy]methyl}propan, das beispielsweise im Handel unter dem Namen Uvinul® 3030 der Fa. BASF AG, Ludwigshafen erhältlich ist.

Zur Gruppe f) der Oxamide zählen beispielsweise 4,4'-Dioctyloxyoxanilid, 2,2'-Diethoxyoxanilid, 2,2'-Dioctyloxy-5,5'-di-tert-butoxanilid, 2,2'-Didodecyloxy-5,5'-di-tert-butoxanilid, 2-Ethoxy-2'-ethyloxanilid, N,N'-Bis(3-dimethylaminopropyl)oxamid, 2-Ethoxy-5-tert-butyl-2'-ethoxanilid und dessen Mischung mit 2-Ethoxy-2'-ethyl-5,4'-di-tert-butoxanilid sowie Mischungen von ortho-, para-Methoxy-disubstituierten Oxaniliden und Mischungen von ortho- und para-Ethoxy disubstituierten Oxaniliden.

Zur Gruppe g) der 2-Phenyl-1,3,5-triazine zählen beispielsweise 2-(2-Hydroxyphenyl)-1,3,5-triazine wie 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propoxy)-phenyl]-4,6-bis(2,4-dimethyl)-1,3,5-triazin, 2-[2-Hydroxy-4-(2-hydroxy-3-octyloxy-propoxy)phenyl]-4,6-bis(2,4-dimethyl)-1,3,5-triazin, 2-[4-(Dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis-(2,4-dimethylphenyl)-1,3,5-triazin, 2-(2-Hydroxy-4-hexyloxyphenyl)-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin und 2-(2-Hydroxyphenyl)-4-(4-methoxyphenyl)-6-phenyl-1,3,5-triazin.

Die Gruppe h) der Antioxidantien umfasst beispielsweise:
Alkylierte Monophenole wie beispielsweise 2,6-Di-tert-butyl-4-methylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Dicyclopentyl-4-methylphenol, 2-(α-Methylcyclohexyl)-4,6-dimethylphenol, 2,6-Dioctadecyl-4-methylphenol, 2,4,6-Tricyclohexylphenol, 2,6-Di-tert-butyl-4-methoxymethylphenol, unverzweigte oder in der Seitenkette verzweigte Nonylphenole wie beispielsweise 2,6-Di-nonyl-4-methylphenol, 2,4-Dimethyl-6-(1-methylundec-1-yl)-phenol, 2,4-Dimethyl-6-(1-methylheptadec-1-yl)-phenol, 2,4-Dimethyl-6-(1-methyltridec-1-yl-)phenol und Gemische davon.

Alkylthiomethylphenole wie zum Beispiel 2,4-Dioctylthiomethyl-6-tert-butylphenol, 2,4-Dioctylthiomethyl-6-methylphenol, 2,4-Dioctylthiomethyl-6-ethylphenol, 2,6-Didodecylthiomethyl-4-nonylphenol.

Hydrochinone und alkylierte Hydrochinone wie zum Beispiel 2,6-Di-tert-butyl-4-methoxyphenol, 2,5-Di-tert-butylhydrochinon, 2,5-Di-tert-amylhydrochinon, 2,6-Diphenyl-4-octadecyloxyphenol, 2,6-Di-tert-butylhydrochinon, 2,5-Di-tert-butyl-4-hydroxyanisol, 3,5-Di-tert-butyl-4-hydroxyanisol, 3,5-Di-tert-butyl-4-hydroxyphenylstearat, Bis-(3,5-di-tert-butyl-4-hydroxyphenyl)adipat.

Tocopherole, wie zum Beispiel α-Tocopherol, β-Tocopherol, γ-Tocopherol, δ-Tocopherol und Gemische davon (Vitamin E).

Hydroxylierte Thiodiphenylether wie zum Beispiel 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis(6-tert-butyl-2-methylphenol), 4,4'-Thio-bis-(3,6-di-sec-amylphenol), 4,4'-Bis(2,6-dimethyl-4-hydroxyphenyl)disulfid.

Alkyliden-Bisphenole wie zum Beispiel 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)butan, 1,1-Bis(5-tert-butyl-4-hydroxy-2-methyl-phenyl)-3-n-dodecylmercaptobutan, Ethylenglycol-bis-[3,3-bis(3-tert-butyl-4-hydroxyphenyl)butyrat], Bis(3-tert-butyl-4-hydroxy-5-methyl-phenyl)dicyclopentadien, Bis[2-(3'-tert-butyl-2-hydroxy-5- methylbenzyl)-6-tert-butyl-4-methylphenyl]terephthalat, 1,1-Bis-(3, 5-dimethyl-2-hydroxyphenyl)butan, 2,2-Bis-(3,5-di-tert-butyl-4-hydroxyphenyl)propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

Benzylverbindungen wie zum Beispiel 3,5,3',5'-Tetra-tert-butyl-4,4'-dihydroxydibenzylether, Octadecyl-4-hydroxy-3,5-dimethylbenzylmercaptoacetat, Tridecyl-4-hydroxy-3,5-di-tert-butylbenzylmercaptoacetat, Tris(3,5-di-tert-butyl-4-hydroxybenzyl)amin, 1,3,5-Tri-(3,5-di-tert-butyl-4-hydroxybenzyl)-2,4,6-trimethylbenzol, Di-(3,5-Di-tert-butyl-4-hydroxybenzyl)sulfid, 3,5-Di-tert-butyl-4-hydroxybenzyl-mercapto-essigsäureisooctylester, Bis-(4-tert-butyl-3-hydroxy-2,6-dimethylbenzyl)dithiolterephthalat, 1,3,5-Tris-(3,5-di-tert-butyl-4-hydroxybenzyl)isocyanurat, 1,3,5-Tris-(4-tert-butyl-3-hydroxy-2, 6-dimethylbenzyl)isocyanurat, 3,5-Di-tert-butyl-4-hydroxybenzyl-phosphorsäuredioctadecylester und 3,5-Di-tert-butyl-4-hydroxybenzyl-phosphorsäuremonoethylester, Calciumsalz.

Hydroxybenzylierte Malonate wie zum Beispiel Dioctadecyl-2,2-bis-(3,5-di-tert butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)malonat, Bis[4-(1,1,3,3-tetramethylbutyl)phenyl]-2,2-bis(3,5-di-tert-butyl-4-hydroxybenzyl)malonat.

Hydroxybenzyl-Aromaten wie zum Beispiel 1,3,5-Tris-(3, 5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)phenol.

Triazinverbindungen wie zum Beispiel 2,4-Bis(octylmercapto)-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.

Benzylphosphonate wie zum Beispiel Dimethyl-2,5-di-tert-butyl-4-hydroxybenzylphosphonat, Diethyl-3,5-di-tert-butyl-4-hydroxybenzylphosphonat ((3,5-Bis(1,1-dimethylethyl)-4-hydroxyphenyl)methyl)Iphosphonsäurediethylester), Dioctade-cyl-3,5-di-tert-butyl-4-hydroxybenzylphosphonat, Dioctadecyl-5-tert-butyl-4-hydroxy-3-methylbenzylphosphonat, Calciumsalz des 3,5-Di-tert-butyl-4-hydroxybenzylphosphonsäure-monoethylesters.

Acylaminophenole wie zum Beispiel 4-Hydroxy-laurinsäureanilid, 4-Hydroxystearinsäureanilid, 2,4-Bis-octylmercapto-6-(3,5-tert-butyl-4-hydroxyanilino)-s-triazin und Octyl-N-(3,5-di-tert-butyl-4-hydroxyphenyl)-carbamat.

Ester der β-(3,5-Di-tert-butyl-4-hydroxyphenyl)propionsäure mit ein- oder mehrwertigen Alkoholen wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(Hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan.

Ester der β-(5-tert-Butyl-4-hydroxy-3-methylphenyl)propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2] octan.

Ester der β-(3,5-Dicyclohexyl-4-hydroxyphenyl)propionsäure mit ein- oder mehrwertigen Alkoholen wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo [2.2.2]octan.

Ester der 3,5-Di-tert-butyl-4-hydroxyphenylessigsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexanediol, Trimethylolpropan, 4-Hydroxymethyl-1-phospha-2,6,7-trioxabi-cyclo[2.2.2]octan.

Amide der β-(3, 5-Di-tert-butyl-4-hydroxyphenyl)propionsäure, wie z. B. N,N'-Bis(3,5-di-tert-butyl-4-hydroxyphenylpropionyl)-hexamethylendiamid, N,N'-Bis(3,5-di-tert-butyl-4-hydroxyphenylpropionyl)-trimethylendiamid, N,N'-Bis(3,5-di-tert-butyl-4-hydroxyphenylpropionyl)-hydrazid, N,N'-Bis[2-(3-[3,5-di-tert-butyl-4-hydroxyphenyl]-propionyloxy)ethyl]-oxamid (z. B. Naugard®XL-1 der Firma Uniroyal).

### Ascorbinsäure (Vitamin C)

Aminische Antioxidantien, wie zum Beispiel N,N'-Di-isopropyl-p-phenylendiamin, N,N'-Di-sec-butyl-p-phenylendiamin, N,N'-Bis(1,4-dimethylpentyl)-p-phenylendiamin, N,N'-Bis(1-ethyl-3-methylpentyl)-p-phenylendiamin, N,N'-Bis(1-methylheptyl)-p-phenylendiamin, N, N'-Dicyclohexyl-p-phenylendiamin, N, N'-Diphenyl-p-phenylendiamin, N,N'-Bis(2-naphthyl)-p-phenylendiamin, N-Isopropyl-N'-phenyl-p-phenylendiamin, N-(1,3-Dimethylbutyl)-N'-phenyl-p-phenylendiamin, N-(1-Methylheptyl)-N'-phenyl-p-phenylendiamin, N-Cyclohexyl-N'-phenyl-p-phenylendiamin, 4-(p-Toluolsulfamoyl)diphenylamin, N,N'-Dimethyl-N,N'-di-sec-butyl-p-phenylendiamin, Diphenylamin, N-Allyldiphenylamin, 4-Isopropoxydiphenylamin, N-Phenyl-1-naphthylamin, N-(4-tert-Octylphenyl)-1-naphthylamin, N-Phenyl-2-naphthylamin, octyliertes Diphenylamin, zum Beispiel p,p'-Di-tert-octyldiphenylamin, 4-n-Butylaminophenol, 4-Butyrylaminophenol, 4-Nonanoylaminophenol, 4-Dodecanoylaminophenol, 4-Octadecanoylaminophenol, Bis-(4-methoxyphenyl)amin, 2,6-Di-tert-butyl-4-dimethylaminomethylphenol, 2,4'-Diaminodiphenylmethan, 4,4'-Diaminodiphenylmethan, N,N,N',N'-Tetramethyl-4,4'-diaminodiphenylmethan, 1,2-Bis-[(2-methylphenyl)amino]ethan,1,2-Bis(phenylamino)-propan, (o-Tolyl)-biguanid, Bis[4-(1',3'-dimethylbutyl)phenyl]amin, tert-octyliertes N-Phenyl-1-naphthylamin, Gemisch aus mono- und dialkylierten tert-Butyl/tert-Octyldiphenylaminen, Gemisch aus mono-und dialkylierten Nonyldiphenylaminen, Gemisch aus mono- und dialkylierten Dodecyldiphenylaminen, Gemisch aus mono- und dialkylierten Isopropyl/Isohexyldiphenylaminen, Gemisch aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, 2,2,6,6-Tetramethylpiperidin-4-on, 2,2,6,6 Tetramethylpiperidin-4-ol, das Dimethylsuccinat-Polymer mit 4-Hydroxy-2,2,6,6-tetramethyl-1-piperidinethanol [CAS Nummer 65447-77-0], (beispielsweise Tinuvin® 622 der Fa. Ciba Specialty Chemicals, Inc.), Polymer of 2,2,4,4-Tetramethyl-7-oxa-3,20-diaza-dispiro[5.1.11.2]-heeicosan-21-on und Epichlorhydrin [CAS-No.: 202483-55-4], beispielsweise (Hostavin® N 30 der Fa. Clariant).

Zu Gruppe i) der Nickelverbindungen gehören zum Beispiel Nickel-Komplexe des 2,2'-Thio-bis-[4-(1,1,3,3-tetramethylbutyl)phenols], wie der 1:1 oder 1:2 Komplex, gegebenenfalls mit zusätzlichen Liganden wie n-Butylamin, Triethanolamin oder N-Cyclohexyldiethanolamin, Nickeldibutyldithiocarbamat, Nickelsalze von 4-Hydroxy-3,5-di-tert-butylbenzylphosphonsäuremonoalkylester wie z. B. der Methyl- oder Ethylester, Nickelkomplexe von Ketoximen wie z. B. von 2-Hydroxy-4-methylphenylundecylketoxim, Nickelkomplex von 1-Phenyl-4-lauroyl-5-hydroxypyrazol, gegebenenfalls mit zusätzlichen Liganden.

Zur Gruppe j) der sterisch gehinderten Amine gehören zum Beispiel 4-Hydroxy-2,2,6,6-tetramethylpiperidin, 1-Allyl-4-hydroxy-2,2,6,6-tetramethylpiperidin, 1-Benzyl-4-hydroxy-2,2,6,6-tetramethylpiperidin, Bis(2,2,6,6-tetramethyl-4-piperidyl)sebacat, Bis(2,2,6,6-tetramethyl-4-piperidyl)succinat, Bis(1,2,2,6,6-pentamethyl-4-piperidyl)sebacat, Bis(1-octyloxy-2, 2,6,6-tetramethyl-4-piperidyl)sebacat, Bis(1,2,2,6,6-pentamethyl-4-piperidyl)-n-butyl-3,5-di-tert-butyl-4-hydroxybenzylmalonat (n-Butyl-3,5-di-tert-butyl-4-hydroxy-benzyl-malonsäure-bis(1,2,2,6,6-pentamethylpiperidyl)-ester), Kondensationsprodukt aus 1-(2-Hydroxyethyl)-2,2,6,6-tetramethyl-4-hydroxypiperidin und Bernsteinsäure, lineare oder cyclische Kondensationsprodukte aus N,N'-Bis(2,2,6,6-tetramethyl-4-piperidyl)hexamethylendiamin und 4-tert-Octylamino-2,6-dichlor-1,3,5-triazin, Tris(2,2,6,6-tetramethyl-4-piperidyl)-nitrilotriacetat, Tetrakis(2,2,6,6-tetramethyl-4-piperidyl)-1,2,3,4-butan-tetracarboxylat, 1,1'-(1, 2-Ethandiyl)-bis(3,3,5,5-tetramethylpiperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert butylbenzyl)malonat, 3-n-Octyl-7,7,9,9-tetramethyl-1,3,8-triazaspiro [4.5]decan-2,4-dion, Bis-(1-octyloxy-2,2,6,6-tetramethylpiperidyl)-sebacat, Bis-(1-octyloxy-2,2,6,6-tetramethyl-piperidyl)-succinat, lineare oder cyclische Kondensationsprodukte von N,N'-Bis-(2,2,6,6-tetramethyl-4-piperidyl)hexamethylendiamin und 4-Morpholino-2,6-dichlor-1,3,5-triazin, Kondensationsprodukt von 2-Chlor-4,6-bis(4-n-butylamino-2, 2,6,6-tetramethylpiperidyl)-1,3,5-triazin und 1,2-Bis(3-aminopropylamino)ethan, Kondensationsprodukt von 2-Chlor-4,6-di-(4-n-butylamino-1,2,2,6,6-pentamethylpiperidyl)-1,3,5-triazin und 1,2-Bis-(3-aminopropylamino)ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)pyrrolidin-2,5-dion, Gemisch von 4-Hexadecyloxy-und 4-Stearyloxy-2,2,6,6-tetramethylpiperidin, Kondensationsprodukt aus N,N'-Bis(2,2,6,6-tetramethyl-4-piperidyl)hexamethylendiamin und 4-Cyclohexylamino-2,6-dichlor-1,3,5-triazin, Kondensationsprodukt aus 1,2-Bis(3-aminopropylamino)ethan und 2,4,6-Trichlor-1,3,5-triazin sowie 4-Butylamino-2,2,6,6-tetramethylpiperidin (CAS Reg. No. [136504-96-6]); N-(2,2,6,6-Tetramethyl-4-piperidyl)-n-dodecylsuccinimid, N-(1,2,2,6,6-Pentamethyl-4-piperidyl)-n-dodecylsuccinimid, 2-Undecyl-7,7,9,9-tetramethyl-1-oxa-3,8-diaza-4-oxospiro[4, 5]decan, Reaktionsprodukt von 7,7,9,9-Tetramethyl-2-cycloundecyl-1-oxa-3,8-diaza-4-oxospiro[4, 5]decan und Epichlorhydrin, 1,1-Bis(1,2,2,6,6-pentamethyl-4-piperidyloxycarbonyl)-2-(4-methoxyphenyl)ethen, Diester der 4-Methoxy-methylenmalonsäure mit 1,2,2,6,6-Pentamethyl-4-hydroxypiperidin, Poly[methylpropyl-3-oxo-4-(2,2,6,6-tetramethyl-4-piperidyl)]siloxan, 1-(2-Hydroxy-2-methylpropoxy)-4-octadecanoyloxy-2,2,6,6-tetramethylpiperidin, 1-(2-Hydroxy-2-methylpropoxy)-4-hexadecanoyloxy-2, 2,6,6-tetramethylpiperidin, das Reaktionsprodukt aus 1-Oxyl-4-hydroxy-2,2,6,6-tetramethylpiperidin und einem Kohlenstoffrest von t-Amylalkohol, 1-(2-Hydroxy-2-methylpropoxy)-4-hydroxy-2,2,6,6-tetramethylpiperidin, 1-(2-Hydroxy-2-methylpropoxy)-4oxo-2,2,6,6-tetramethylpiperidin, Bis(1-(2-hydroxy-2-methylpropoxy)-2,2,6,6-tetramethylpiperidin-4-yl)sebacat, Bis(1-(2-hydroxy-2-methylpropoxy)-2, 2,6,6-tetramethylpiperidin-4-yl)adipat, Bis(1-(2-hydroxy-2-methylpropoxy)-2, 2,6,6-tetramethylpiperidin-4-yl)succinat, Bis(1-(2-hydroxy-2-methylpropoxy)-2, 2,6,6-tetramethylpiperidin-4-yl)glutarat, 2,4-bis{N[1-(2-hydroxy-2-methylpropoxy)-2,2,6,6-tetramethylpiperidin-4-yl]-N-butylamino}-6-(2hydroxyethylamino)-s-triazin, Hexahydro-2,6-bis(2,2,6,6-tetramethyl-4-piperidyl)-1 H,4H,5H,8H-2,3a,4a,6,7a,8a-hexaazacyclopenta[def]fluoren-4,8-dion (z. B. Uvinul® 4049 der Fa. BASF AG, Ludwigshafen), Poly[[6-[(1,1,3,3-tetramethylbutyl)amino]-1,3,5-triazin-2,4-diyl][(2,2,6,6-tetramethyl-4-piperidinyl)imino]1,6-hexandiyl[(2,2,6,6-tetramethyl-4-piperidinyl)imino]]) [CAS Nr. 71878-19-8], 1,3,5-Triazin-2,4,6-triamin,N,N"'-[1,2-ethan-diyl-bis [[4,6-bis-[butyl(1,2,2,6,6-pentamethyl-4-piperidinyl)amino]-1,3,5-triazin-2-yl]imino]-3,1-propandiyl]]bis[N',N"-dibutyl-N',N"-bis(1,2,2,6,6-pentamethyl-4-piperidinyl)- (CAS Nr. 106990-43-6) (z. B. Chimassorb 119 der Fa. Ciba Specialty Chemicals, Inc.).

Zur Gruppe k) der Metalldesaktivatoren gehören zum Beispiel N,N'-Diphenyloxalsäurediamid, N-Salicylal-N'-salicyloyl-hydrazin, N,N'-Bis(salicyloyl)hydrazin, N, N'-Bis(3,5-di-tert-butyl-4-hydroxyphenylpropionyl)hydrazin, 3-Salicyloylamino-1,2,4-triazol, Bis(benzyliden)-oxalyldihydrazid, Oxanilid, Isophthaloyldihydrazid, Sebacoylbisphenylhydrazid, N, N'-Diacetyladipinsäuredihydrazid, N,N'-Bis(salicyloyl)oxalsäuredihydrazid, N,N'-Bis(salicyloyl)thiopropionyldihydrazid.

Zur Gruppe I) der Phosphite und Phosphonite gehören zum Beispiel Triphenylphosphit, Diphenylalkylphosphite, Phenyldialkylphosphite, Tris(nonylphenyl)phosphit, Trilaurylphosphit, Trioctadecylphosphit, Distearylpentaerythritdiphosphit, Tris(2,4-di-tert-butylphenyl)phosphit, Diisodecylpentaerythritdiphosphit, Bis(2,4-di-tert-butylphenyl)pentaerythritdiphosphit, Bis(2,6-di-tert-butyl-4-methylphenyl)-pentaerythritdiphosphit, Diisodecyloxypentaerythritdiphosphit, Bis(2,4-di-tert-butyl-6-methylphenyl)-pentaerythritdiphosphit, Bis(2,4,6-tris(tert-butylphenyl)pentaerythrit-diphosphit, Tristearylsorbittriphosphit, Tetrakis-(2,4-di-tert-butylphenyl)-4,4'-biphenylendiphosphonit, 6-Isooctyloxy-2,4,8,10-tetra-tert-butyl-dibenz[d,f][1,3,2]di-oxaphosphepin, 6-Fluor-2,4,8,10-tetra-tert-butyl-12-methyl-dibenz[d,g][1,3,2]di-oxaphosphocin, Bis(2,4-di-tert-butyl-6-methylphenyl)methylphosphit, Bis(2,4-di-tert-butyl-6-methylphenyl)ethylphosphit, 2,2',2"-Nitrilo[triethyl-tris(3,3', 5,5'-tetra-tert-butyl-1,1'-biphenyl-2,2'-diyl)phosphit], 2- Ethylhexyl-(3,3', 5,5'-tetra-tert-butyl-1,1'-biphenyl-2,2'-diyl)phosphit .

Zur Gruppe m) der Hydroxylamine gehören zum Beispiel N, N-Dibenzylhydroxylamin, N, N-Diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecyl-hydroxylamin, N-Hexadecyl-N-octadecylhydroxylamin, N-Heptadecyl-N-octadecylhydroxylamin, N-Methyl-N-octadecylhydroxylamin und N, N-Dialkylhydroxylamin aus hydrierten Talgfettaminen.

Zur Gruppe n) der Nitrone gehören zum Beispiel N-Benzyl-α-phenylnitron, N-Ethyl-αmethylnitron, N-Octyl-α-heptylnitron, N-Lauryl-α-undecylnitron, N-Tetradecyl-αtridecylnitron, N-Hexadecyl-α-pentadecylnitron, N-Octadecyl-α-heptadecylnitron, N-Hexadecyl-α-heptadecylnitron, N-Ocatadecyl-α-pentadecylnitron, N-Heptadecyl-αheptadecylnitron, N-Octadecyl-α-hexadecylnitron, N-Methyl-α-heptadecylnitron und Nitrone, abgeleitet von N,N-Dialkylhydroxylaminen hergestellt aus hydrierten Talkfettaminen.

Zur Gruppe o) der Aminoxide gehören zum Beispiel Aminoxidderivate wie sie in den U.S. Patenten Nr. 5,844,029 und 5,880,191 beschrieben sind, Didecylmethylaminoxid, Tridecylaminoxid, Tridodecylaminoxid und Trihexadecylaminoxid.

Zur Gruppe p) der Benzofuranone und Indolinone gehören zum Beispiel die in den US-Patenten 4,325,863; 4,338,244; 5,175,312; 5,216,052; 5,252,643; in der DE-A-4316611; in der DE-A-4316622; in der DE-A-4316876; in der EP-A-0589839 oder EP-A-0591102 beschriebenen oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]benzofuran-2-on, 3,3'-Bis[5,7-di-tert-butyl-3-(4-[2-hydroxyethoxy]phenyl)benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxyphenyl)-5,7-di-tert-butyl-benzofuran-2-on, 3-(3,4-Dimethylphenyl)-5,7-di-tert-butyl-benzofuran-2-on, Irganoxs HP-136 der Firma Ciba Specialty Chemicals, und 3-(2,3-Dimethylphenyl)-5,7-di-tert-butyl-benzofuran-2-on.

Zur Gruppe q) der Thiosynergisten gehören zum Beispiel Dilaurylthiodipropionat oder Distearylthiodipropionat.

Zur Gruppe r) der peroxidzerstörende Verbindungen gehören zum Beispiel Ester der β-Thiodipropionsäure, zum Beispiel der Lauryl-, Stearyl-, Myristyl- oder Tridecylester, Mercaptobenzimidazol oder das Zinksalz des 2-Mercaptobenzimidazols, Zinkdibutyldithiocarbamat, Dioctadecyldisulfid, Pentaerythrit-tetrakis(β-dodecylmercapto)-propionat.

Zur Gruppe s) der basischen Costabilisatoren gehören zum Beispiel Melamin, Polyvinylpyrrolidon, Dicyandiamid, Triallylcyanurat, Harnstoffderivate, Hydrazinderivative, Amine, Polyamide, Polyurethane, Alkali- und Erdalkalisalze höherer Fettsäuren, zum Beispiel Calciumstearat, Zinkstearat, Magnesiumbehenat, Magnesiumstearat, Natriumricinoleat und Kaliumpalmitat, Antimonbrenzcatechinat oder Zinkbrenzcatechinat.

Weiterhin kann die erfindungsgemäße Mischung als Komponente (d) oder der Kunststoff sonstige Additive und Zusatzstoffe enthalten. Als geeignete Additive der Gruppe t) kommen die üblichen Zusatzstoffe, wie z. B., Pigmente, Farbstoffe, Nukleierungsmittel, Füll- oder Verstärkungsmittel, Beschlagverhinderungsmittel, Biozide und Antistatika, in Betracht.

Geeignete Pigmente sind anorganische Pigmente, beispielsweise Titandioxid in seinen drei Modifikationen Rutil, Anatas oder Brookit, Ultramarinblau, Eisenoxide, Bismutvanadate oder Ruß sowie die Klasse der organischen Pigmente, beispielsweise Verbindungen aus der Klasse der Phthalocyanine, Perylene, Azoverbindungen, Isoindoline, Chinophthalone, Diketopyrolopyrole, Chinacridone, Dioxazine, Indanthrone.

Unter Farbstoffen sind alle Farbmittel zu verstehen, die sich im verwendeten Kunststoff vollständig lösen bzw. in einer molekulardispersen Verteilung vorliegen und somit zur hochtransparenten, nichtstreuenden Einfärbung von Polymeren verwendet werden können. Ebenfalls als Farbstoffe sind organische Verbindungen anzusehen, die eine Fluoreszenz im sichtbaren Teil des elektromagnetischen Spektrums aufweisen wie Fluoreszenzfarbstoffe.

Geeignete Nukleierungsmittel umfassen zum Beispiel anorganische Stoffe, beispielsweise Talk, Metalloxide wie Titandioxid oder Magnesiumoxid, Phosphate, Carbonate oder Sulfate von vorzugsweise Erdalkalimetallen; organische Verbindungen wie Mono-oder Polycarbonsäuren sowie ihre Salze wie z. B. 4-tert-Butylbenzoesäure, Adipinsäure, Diphenylessigsäure, Natriumsuccinat oder Natriumbenzoat; polymere Verbindungen, wie beispielsweise ionische Copolymerisate ("Ionomere").

Geeignete Füll- oder Verstärkungsstoffe umfassen zum Beispiel Calciumcarbonat, Silikate, Talk, Mica, Kaolin, Glimmer, Bariumsulfat, Metalloxide und -hydroxide, Ruß, Graphit, Holzmehl und Mehle oder Fasern anderer Naturprodukte, synthetische Fasern. Als Beispiele für faserförmige bzw. pulverförmige Füllstoffe kommen außerdem Kohlenstoff- oder Glasfasern in Form von Glasgeweben, Glasmatten oder Glasseidenrovings, Schnittglas, Glaskugeln sowie Wollstonit in Betracht. Die Einarbeitung von Glasfasern kann sowohl in Form von Kurzglasfasern als auch in Form von Endlosfasern (Rovings) erfolgen.

Geeignete Antistatika sind beispielsweise Aminderivate wie N,N-Bis(hydroxyalkyl)alkylamine oder -alkylenamine, Polyethylenglycolester und -ether, ethoxylierte Carbonsäureester- und -amide und Glycerinmono- und - distearate, sowie deren Mischungen.

Als Kunststoffe, die durch die erfindungsgemäßen Mischungen stabilisiert werden können, seien beispielsweise genannt:

### Thermoplastische Elastomere;

Polymere von Mono- und Diolefinen, wie zum Beispiel Polyethylen niedriger und hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie zum Beispiel Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol sowie Copolymere von Styrol oder α-Methylstyrol mit Dienen und/oder Acrylderivaten, wie zum Beispiel Styrol-Butadien, Styrol-Acrylnitril (SAN), Styrol-Ethylmethacrylat, Styrol-Butadien-Ethylacrylat, Styrol-Acrylnitril-Methacrylat, AcrylnitrilButadien-Styrol (ABS) oder Methylmethacrylat-Butadien-Styrol (MBS);
Halogenhaltige Polymere, wie zum Beispiel Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, zum Beispiel Polyvinylalkohol und Polyvinylacetat;

Polyurethane, Polyamide, Polyharnstoffe, Polyester, Polycarbonate, Polysulfone, Polyethersulfone und Polyetherketone.

Weiterhin können mit den erfindungsgemäßen Mischungen Lacküberzüge stabilisiert werden, zum Beispiel Industrielackierungen. Unter diesen sind Einbrennlackierungen, unter diesen wiederum Fahrzeuglackierungen, vorzugsweise Zweischichtlackierungen, besonders hervorzuheben. Ein anderes Verwendungsgebiet sind zum Beispiel Anstrichmittel für Außenanstriche von Gebäuden, anderen Bauwerken oder technischen Apparaturen.

Die erfindungsgemäßen Mischungen können in fester oder gelöster Form dem Lack zugesetzt werden. Ihre gute Löslichkeit in Lacksystemen ist dabei von besonderem Vorteil.

Bevorzugt werden die erfindungsgemäßen Mischungen zum Stabilisieren von thermoplastischen Elastomeren eingesetzt, zum Beispiel auf Polyolefinbasis. Insbesondere finden die erfindungsgemäßen Mischungen Einsatz in der Stabilisierung von Formmassen aus den genannten Materialien.

Ein weiteres bevorzugtes Einsatzgebiet ist die Stabilisierung von Polyethylen niedriger und hoher Dichte, sowie von Polypropylen und Polyamid, beispielsweise auch von Fasern hieraus.

Das erfindungsgemäßen Mischungen zeigen eine verbesserte Stabilisierung unbelebter organischer Materie gegenüber Licht mit einem hohen UV-Anteil und/oder hoher Lichtintensität. Weiterhin beruhen die erfindungsgemäßen Mischungen auf einfach zugänglichen Ausgangsstoffen. Mit Hilfe der erfindungsgemäßen Mischungen ist es möglich einen effizienten Schutz unbelebter organischer Materie gegenüber Sauerstoff oder Wärme zu gewährleisten.
Die vorstehenden Ausführungsformen des erfindungsgemäßen Verfahrens und die nachfolgenden Beispiele verdeutlichen beispielhaft die vorliegende Erfindung. Es sind jedoch viele weitere Variationen des Verfahrens und Kombinationen der Merkmale des erfindungsgemäßen Verfahrens für den Fachmann denkbar, ohne den Rahmen der Patentansprüche zu verlassen.

### Beispiele

TPO-Plättchen basieren auf thermoplastischem Elastomer auf Polyolefinbasis (Polypropylen, AW161C, "reactor grade" TPO der Firma Sumitomo Chemical Industries Co., Ltd.)

### Beispiel 1: Herstellung erfindungsgemäßer Mischungen

1a) 100 g Poly-{3-(eicosyl-tetracosyl)-1-[2,2,6,6-tetramethylpiperidin-4-yl]-pyrrolidin-2,5-dion} (CAS Nr. 152261-33-1; HALS Ia) wurden durch Erwärmen geschmolzen und mit 50 g Hexadecyl-3,5-di-tert-butyl-4-hydroxybenzoat (IIIa) versetzt. Nach der Homogenisierung wurden aus der heißen Mischung Pastillen geformt, die beim Erkalten erstarrten.
1b) 40 g N,N'-Bisformyl-N,N'-bis-(2,2,6,6-tetramethyl-4-piperidinyl)-hexamethylendiamin (IIa) und 20 g 2,4-Di-tert-butyl-phenyl-3,5-Di-tert-butyl-4-hydroxybenzoat wurden in einem Taumelmischer zusammengegeben und innig vermengt.

### Beispiel 2: Synergistische Wirkung der Mischung gegenüber den Einzelkomponenten

Beige eingefärbte TPO-Plättchen mit verschiedenen Stabilisatoren wurden hergestellt und anschließend gemäß SAE J 1885 bewittert. Die Farbänderung (ΔE) wurde nach einer Bestrahlung von 450 MJ/m² gemessen. Die Ergebnisse sind in den folgenden Tabellen dargestellt (je geringer die Farbänderung ausfällt, desto besser sind die Resultate):

| | Stabilisierung | ΔE nach 450 MJ/m² |
|---|---|---|
| 1 | 0.15% HALS la | 5,3 |
| 2 | 0.1% HALS la | 4,4 |
| | 0.05% Benzoat IIIb | |
| | | |

| | Stabilisierung | ΔE nach 450 MJ/m² |
|---|---|---|
| 1 | 0.075% HALS Ia | 3,9 |
| | 0,075% HALS IIa | |
| 2 | 0.05% HALS Ia | 0,2 |
| | 0.05% HALS IIa | |
| | 0,05% Benzoat IIIa | |

Anhand der Ergebnisse ist zu erkennen, dass die erfindungsgemäßen Mischungen (jeweils Nr. 2) bessere Resultate zeigen, als die entsprechenden Vergleichsversuche (jeweils Nr. 1) mit (Mischungen von) HALS-Verbindungen.

### Beispiel 3: Glanzerhalt von eingefärbten TPO-Plättchen

Beige eingefärbte TPO-Plättchen mit verschiedenen Stabilisatoren wurden hergestellt und anschließend gemäß SAE J 1885 bewittert. Der Glanzerhalt (unter 60°-Winkel gemessen) wurde nach einer Bestrahlung von 150 und 300 MJ/m² gemessen. Der Glanz ist ein Maß für die Glätte der Oberflächen. Bei Schädigung der Oberfläche wird diese rau, und der Glanzwert fällt ab. Die Ergebnisse sind in der folgenden Tabelle dargestellt:

| | Stabilisierung | Glanz (60 °), Referenz | Glanz (60 °) nach 150 MJ/m² | Glanz (60 °) nach 300 MJ/m² |
|---|---|---|---|---|
| 1 | 0.1% HALS Ia | 72,2 | 51,8 | 4,8 |
| | 0.05% Benzoat IIIb | | | |
| 2 | 0.1% HALS IIa | 69,5 | 62,7 | 55,2 |
| | 0.05% Benzoat IIIb | | | |
| 3 | 0.05% HALS Ia | 73,9 | 57,6 | 48,8 |
| | 0.05% HALS IIa | | | |
| | 0.05% Benzoat IIIb | | | |
| 4 | 0.1 % HALS IV* | 70,4 | 13,0 | 2,3 |
| | 0.05% Benzoat IIIb | | | |
| 5 | 0,1% HALS V* | 71,7 | 7,1 | 2,3 |
| | 0,05% Benzoat IIIb | | | |

| | | | | |
|---|---|---|---|---|
| *) HALS IV ist Poly[[6-[(1,1,3,3-tetramethylbutyl)amino]-1,3,5-triazin-2,4-diyl][(2,2,6,6-tetramethyl-4-piperidinyl)imino]1,6-hexandiyl[(2,2,6,6-tetramethyl-4-piperidinyl)imino]]) (CAS Nr. 71878-19-8, Chimassorb 944); HALS V ist N,N"'-[1,2-ethane-diyl-bis [[[4,6-bis-[butyl (1,2,2,6,6-pentamethyl-4-piperidinyl)amino]-1,3,5-triazine-2-yl] imino]-3,1-propanediyl]] bis [N',N"- dibutyl-N',N"-bis(1,2,2,6,6-pentamethyl-4-piperidinyl)-1,3,5-Triazine-2,4,6-triamine (CAS-Nr. 106990-43-6, Chimassorb 199) | | | | |

Die vorliegenden Ergebnisse zeigen deutlich, dass die erfindungsgemäßen Mischungen von HALS und Benzoat (Nr. 1-3) eine gute Stabilisierungswirkung gegenüber den Vergleichsversuchen mit HALS IV (Nr. 4) bzw. HALS V (Nr. 5) aufweisen.

## Patentansprüche

1. Mischungen, enthaltend
(a) eine oligomere Verbindung, enthaltend Wiederholeinheiten der allgemeinen Formel (I) oder deren Säureadditionssalze, 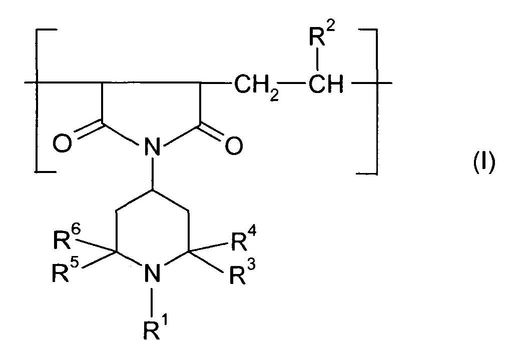 worin,
R¹ H, C₁-C₃₀-Alkyl, C₂-C₂₂-Alkenyl, C₁-C₂₀-Alkoxy, Cyanomethyl, 2-Hydroxyethyl, Formyl, C₂-C₆-Alkanoyl, Benzyl oder einen Rest der Formel -CR⁷=CH-CO-OR⁸
R² H, C₁-C₃₀-Alkyl, Mischung aus C₁₄- bis C₂₈-Alkylgruppen,
R³, R⁴, R⁵, R⁶ unabhängig voneinander, gleich oder verschieden, C₁-C₃₀-Alkyl,
R⁷ H, C₁-C₆-Alkyl, oder einen Rest der Formel CO-OR⁸,
R⁸ C₁-C₁₈-Alkyl, C₃-C₁₅-Cycloakyl, C₇-C₁₈-Aralkyl, Phenyl oder Tolyl,
bedeuten,
und/oder
(b) eine Verbindung der allgemeinen Formel (II) oder deren Säureadditionssalze, 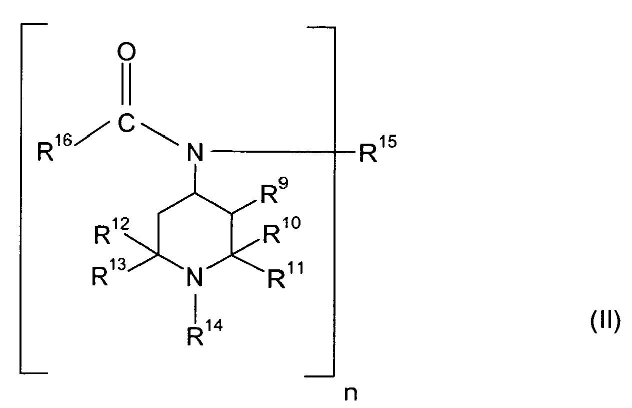 worin,
n 1 oder 2,
R⁹ H, C₁-C₄-Alkyl,
R¹⁰, R¹¹, R¹², R¹³ unabhängig voneinander, gleich oder verschieden, C₁-C₄-Alkyl oder R¹⁰ und R¹¹ oder R¹² und R¹³ zusammen eine Tetramethylen- oder Pentamethylengruppe,
R¹⁴ H, C₁-C₃₀-Alkyl, C₂- C₂₂-Alkenyl, gegebenenfalls durch C₁-C₄-Alkyl, Halogen, C₁-C₄-Alkoxy, Methylendioxy, Ethy- lendioxy und/oder Di-C₁-C₄-alkylamino substituiertes C₇- C₁₂-Phenylalkyl, C₁-C₂₂-Alkanoyl, C₂-C₃-Cyanalkyl, C₁- C₂₂-Hydroxyalkyl oder C₂-C₂₂-Aminoalkyl
R¹⁶ H, C₁-C₃₀-Alkyl,
und
- wenn n = 1 ist -
R¹⁵ H, C₁-C₂₂-Alkyl, C₂-C₂₂-Alkenyl, C₃-C₁₂-Cycloalkyl oder Bicycloalkyl, durch Cyan, Hydroxy oder Carbo-C₁-C₄-alkoxy substituiertes C₂-C₂₂-Alkyl, durch Ethersauerstoff, Stickstoff oder Schwefel unterbrochenes C₄-C₂₂-Alkyl, gegebenenfalls durch C₁-C₄-Alkyl, Halogen, C₁-C₄-Alkoxy, Methylendioxy, Ethy- lendioxy oder Di-C₁-C₄-alkylamino substituiertes C₇-C₂₂-Phenyl- oder Diphenylalkyl, gegebenenfalls durch C₁-C₄-Alkyl oder Carbo-C₁-C₄-alkoxy substituiertes Phe- nyl, ein Rest der allgemeinen Formel (IV) 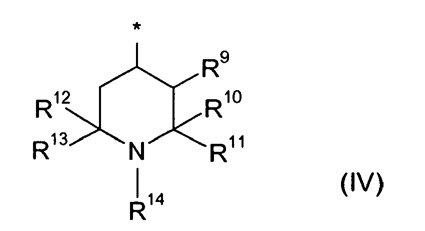 oder heterocyclische Reste enthaltendes C₁-C₂₂-Alkyl,
oder
- wenn n = 2 ist -
R¹⁵ C₂-C₂₂-Alkylen, C₅-C₂₂-Cycloalkylen, C₈-C₁₄-Phenylalkylen, Phenylen oder durch Ethersauerstoff, Stickstoff, Schwefel oder 5 - oder 6-gliedrige Heterocyclen unterbrochenes C₄-C₃₀-Alkylen
bedeuten,
und
(c) wenigstens eine Verbindung der allgemeinen Formel (III), 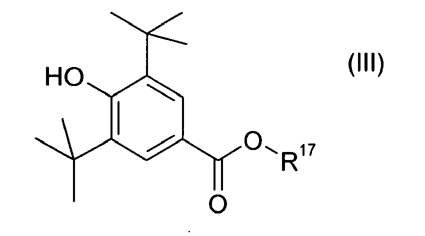 worin,
R¹⁷ C₁-C₂₀-Alkyl, Aryl,
bedeutet,
und
(d) optional weitere Zusatzstoffe,
wobei,
die Substituenten R¹ bis R¹⁷ jeweils an beliebiger Position durch ein oder mehrere Heteroatome unterbrochen sein können, wobei die Anzahl dieser Heteroatome nicht mehr als 10 beträgt, und/oder jeweils an beliebiger Position, allerdings nicht mehr als fünfmal, durch C₁-C₃₀-Alkyl, C₁-C₂₀-Alkoxy, Aryl, Heterocyclen, Heteroatome oder Halogen substituiert sein können, wobei diese ebenfalls maximal zweimal, bevorzugt maximal einmal mit den genannten Gruppen oder substituierten Arylen substituiert sein können.

2. Mischung nach Anspruch 1, wobei zusätzlich als Komponente (d) Antioxidantien enthalten sind.

3. Mischung nach Anspruch 1 oder 2, mit R² entsprechend einer Mischung aus C₁₄-bis C₂₈-Alkylgruppen, wobei zwei dieser Alkylgruppen, die sich um nicht mehr als zwei C-Atome unterscheiden dürfen, jeweils mindestens 30 % dieser Mischung ausmachen.

4. Mischung nach den Ansprüchen 1 bis 3, wobei Komponente (a) eine oligomere Verbindung mit einer Wiederholeinheit der allgemeinen Formel (I) mit R³, R⁴, R⁵ und R⁶ = CH₃ und R² = C₁₆-C₂₆-Alkyl enthält.

5. Mischung nach den Ansprüchen 1 bis 4, wobei Komponente (b) eine Verbindung der allgemeinen Formel (II) enthält, wobei R³, R⁴, R⁵ und R⁶ für Methyl und R² für ein Wasserstoffatom oder C₂-C₁₀-Alkyl steht.

6. Mischung nach den Ansprüchen 1 bis 5, wobei Komponente (c) eine Verbindung der allgemeinen Formel (III) mit R¹⁷=C₁₆-Alkyl enthält.

7. Mischung nach den Ansprüchen 1 bis 6, wobei Komponente (c) eine Verbindung der allgemeinen Formel (III) mit R¹⁷ = 2,4 Di-t-butyl-phenyl enthält.

8. Mischung nach den Ansprüchen 1 bis 7, wobei das Gewichtsverhältnis der Komponenten (a) und/oder (b) zu der Komponente (c) zwischen 10:1 und 1:2 liegt.

9. Verwendung der Mischung gemäß den Ansprüchen 1 bis 8 zur Stabilisierung von unbelebten organischen Materialien gegen die Einwirkung von Licht, Sauerstoff und/oder Wärme.

10. Verwendung nach Anspruch 9 zur Stabilisierung von Kunststoffen, bestehend aus mindestens einem Polymer, ausgewählt aus thermoplastischen Elastomeren, Polyolefinen, Polstyrol, Copolymeren von Styrol oder α-Methylstyrols, Polyestern, Polycarbonaten, Polyvinylchlorid, Polyacrylaten, Polymethacrylaten, Polyurethanen sowie physikalischen Blends der zuvor genannten Polymere.

11. Verwendung nach Anspruch 10 zur Stabilisierung von thermoplastischen Elastomeren auf Olefinbasis.

12. Unbelebte organische Materialien, enthaltend wenigstens eine Mischung gemäß den Ansprüchen 1 bis 8.

13. Gegenstände, hergestellt aus unbelebten organischen Materialien gemäß Anspruch 12.

14. Verfahren zur Stabilisierung von unbelebten organischen Materialien, gegen die Einwirkung von Licht, Sauerstoff und/oder Wärme, **dadurch gekennzeichnet, dass** man den unbelebten organischen Materialien mindestens eine Mischung gemäß den Ansprüchen 1 bis 8 in einer effektiven Menge zugibt.

## Claims

1. A mixture, comprising
(a) an oligomeric compound, comprising repeat units of the general formula (I) or acid-addition salts thereof 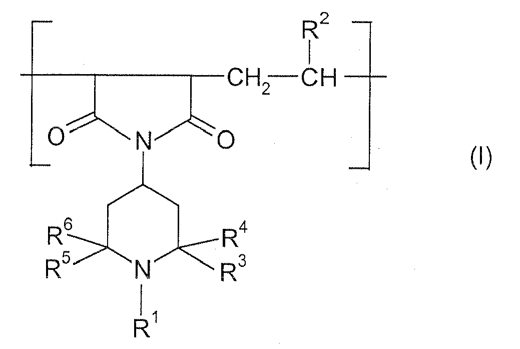 in which
R¹ is H, C₁-C₃₀-alkyl, C₂-C₂₂-alkenyl, C₁-C₂₀-alkoxy, cyanomethyl, 2-hydroxyethyl, formyl, C₂-C₆-alkanoyl, benzyl, or a moiety of the formula -CR⁷=CH-CO-OR⁸,
R² is H, C₁-C₃₀-alkyl, or a mixture composed of C₁₄-C₂₈-alkyl groups,
R³, R⁴, R⁵, and R⁶, independently of one another, are identical or different C₁-C₃₀-alkyl,
R⁷ is H, C₁-C₆-alkyl, or a moiety of the formula CO-OR⁸,
R⁸ is C₁-C₁₈-alkyl, C₃-C₁₅-cycloakyl, C₇-C₁₈-aralkyl, phenyl or tolyl,
and/or
(b) a compound of the general formula (II) or acid-addition salts thereof 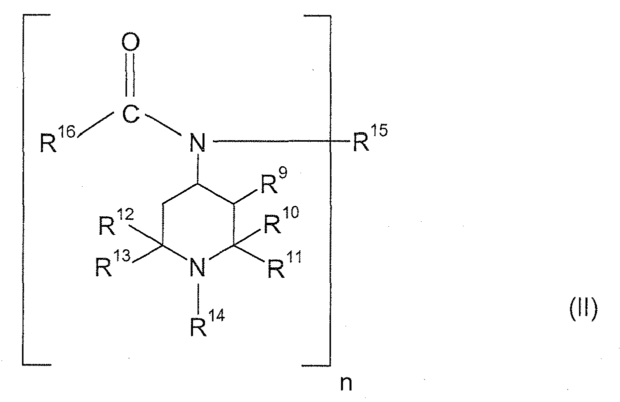 in which
n is 1 or 2,
R9 is H or C₁-C₄-alkyl,
R¹⁰, R¹¹, R¹², and R¹³, independently of one another, are identical or different C₁- C₄-alkyl, or R¹⁰ and R¹¹ or R¹² and R¹³ together are a tetramethylene group or pentamethylene group,
R¹⁴ is H, C₁-C₃₀-alkyl, or C₂- C₂₂-alkenyl, or unsubstituted or C₁-C₄-alkyl-, halogen-, C₁-C₄-alkoxy-, methylenedioxy-, ethylenedioxy-, and/or di-C₁-C₄-alkylamino-substituted C₇- C₁₂-phenylalkyl, C₁-C₂₂-alkanoyl, C₂-C₃-cyanoalkyl, C₁- C₂₂-hydroxyalkyl or C₂-C₂₂-aminoalkyl,
R¹⁶ is H or C₁-C₃₀-alkyl,
and
- if n = 1 -
R¹⁵ is H, C₁-C₂₂-alkyl, C₂-C₂₂-alkenyl, C₃-C₁₂-cycloalkyl or bicycloalkyl, or cyano-, hydroxyl-, or carbo-C₁-C₄- alkoxy-substituted C₂-C₂₂-alkyl, or ether-oxygen-, nitrogen-, or sulfur-interrupted C₄-C₂₂-alkyl, or unsubstituted or C₁-C₄-alkyl-, halogen-, C₁-C₄-alkoxy-, methylenedioxy-, ethylenedioxy-, or di-C₁-C₄-alkylamino- substituted C₇-C₂₂-phenyl- or diphenylalkyl, or unsubstituted or C₁-C₄-alkyl- or carbo-C₁-C₄- alkoxy-substituted phenyl, or a moiety of the general formula (IV) 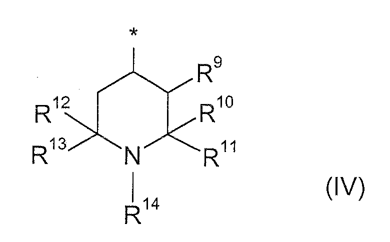 or C₁-C₂₂-alkyl comprising heterocyclic moieties,
or
- if n = 2 -
R¹⁵ is C₂-C₂₂-alkylene, C₅-C₂₂-cycloalkylene, C₈-C₁₄-phenylalkylene, or phenylene, or ether-oxygen-, nitrogen-, or sulfur-interrupted C₄-C₃₀-alkylene, or C₄-C₃₀-alkylene interrupted by 5- or 6- membered heterocyclic moieties,
and
(c) at least one compound of the general formula (III) 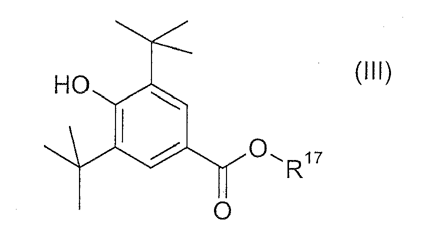 in which
R¹⁷ is C₁-C₂₀-alkyl or aryl,
and
(d) optionally further additives,
where
each of the substituents R¹ to R¹⁷ can have interruption at any desired position by one or more heteroatoms, where the number of these heteroatoms is not more than 10, and/or each of the substituents R¹ to R¹⁷ can have substitution at any desired position, but not more than five times, by C₁-C₃₀-alkyl, C₁-C₂₀-alkoxy, aryl, heterocyclic moieties, heteroatoms, or halogen, and these likewise can have substitution at most twice, preferably at most once by the groups mentioned or by substituted aryl moieties.

2. The mixture according to claim 1, where antioxidants are additionally comprised, as component (d).

3. The mixture according to claim 1 or 2, where R² corresponds to a mixture composed of C₁₄-C₂₈-alkyl groups, where two of these alkyl groups which are not permitted to differ from one another by more than two carbon atoms make up respectively at least 30% of this mixture.

4. The mixture according to claims 1 to 3, where component (a) comprises an oligomeric compound having a repeat unit of the general formula (I), where R³,R⁴, R⁵ and R⁶ = CH₃ and R² = C₁₆-C₂₆-alkyl.

5. The mixture according to claims 1 to 4, where component (b) comprises a compound of the general formula (II), where R³,R⁴, R⁵ and R⁶ are methyl, and R² is a hydrogen atom or C₂-C₁₀-alkyl.

6. The mixture according to claims 1 to 5, where component (c) comprises a compound of the general formula (III), where R¹⁷ = C₁₆-alkyl.

7. The mixture according to claims 1 to 6, where component (c) comprises a compound of the general formula (III), where R¹⁷ = 2,4-di-tert-butylphenyl.

8. The mixture according to claims 1 to 7, where the ratio by weight of components (a) and/or (b) to component (c) is from 10:1 to 1:2.

9. The use of the mixture according to claims 1 to 8 for the stabilization of non-living organic materials with respect to exposure to light, oxygen, and/or heat.

10. The use according to claim 9 for the stabilization of plastics, composed of at least one polymer, selected from thermoplastic elastomers, polyolefins, polystyrene, copolymers of styrene or of α-methylstyrene, polyesters, polycarbonates, polyvinyl chloride, polyacrylates, polymethacrylates, polyurethanes, and also physical blends of the abovementioned polymers.

11. The use according to claim 10 for the stabilization of olefin-based thermoplastic elastomers.

12. A non-living organic material, comprising at least one mixture according to claims 1 to 8.

13. An particle, produced from non-living organic materials according to claim 12.

14. A process for the stabilization of non-living organic materials with respect to exposure to light, oxygen, and/or heat, which comprises adding an effective amount of at least one mixture according to claims 1 to 8 to the non-living organic materials.

## Revendications

1. Mélanges, contenant
(a) un composé oligomère, contenant des unités de répétition de formule générale (I), ou ses sels d'addition acide 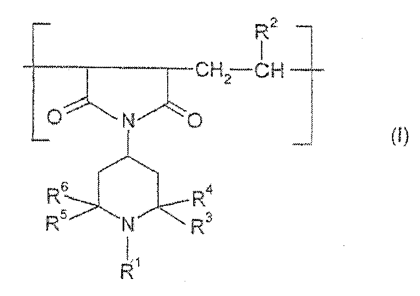 dans laquelle
R¹ signifie H, alkyle en C₁-C₃₀, alcényle en C₂-C₂₂, alcoxy en C₁-C₂₀, cyanométhyle, 2-hydroxyéthyle, formyle, alcanoyle en C₂-C₆, benzyle ou un radical de formule -CR⁷=CH-CO-OR⁸
R² signifie H, alkyle en C₁-C₃₀, mélange de groupes alkyle en C₁₄ à C₂₈,
R³, R^{4'} R⁵, R⁶ sont identiques ou différents et signifient, indépendamment les uns des autres, alkyle en C₁-C₃₀,
R⁷ signifie H, alkyle en C₁-C₆ ou un radical de formule CO-OR⁸,
R⁸ signifie alkyle en C₁-C₁₈, cycloalkyle en C₃-C₁₅, aralkyle en C₇-C₁₈, phényle ou tolyle,
et/ou
(b) un composé de formule générale (II) ou ses sels d'addition acide, 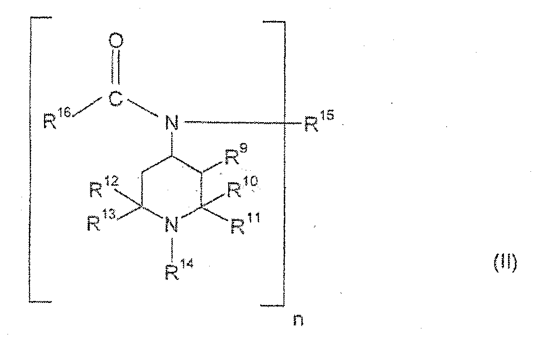 dans laquelle
n signifie 1 ou 2,
R⁹ signifie H, alkyle en C₁-C₄,
R¹⁰, R¹¹, R¹², R¹³ sont identiques ou différents et signifient, indépendamment les uns des autres, alkyle en C₁-C₄, ou R¹⁰ et R¹¹ ou R¹² et R¹³ forment ensemble un groupe tétraméthylène ou pentaméthylène,
R¹⁴ signifie H, alkyle en C₁-C₃₀, alcényle en C₂-C₂₂, phénylalkyle en C₇-C₁₂ éventuellement substitué par alkyle en C₁-C₄, halogène, alcoxy en C₁-C₄, méthylènedioxy, éthylènedioxy et/ou dialkylamino en C₁-C₄, alcanoyle en C₁-C₂₂, cyanalkyle en C₂-C₃, hydroxyalkyle en C₁-C₂₂ ou aminoalkyle en C₂-C₂₂,
R¹⁶ signifie H, alkyle en C₁-C₃₀,
et
- lorsque n = 1,
R¹⁵ signifie H, alkyle en C₁-C₂₂, alcényle en C₂-C₂₂, cycloalkyle ou bicycloalkyle en C₃-C₁₂, alkyle en C₂-C₂₂ substitué par cyano, hydroxy ou carboalcoxy en C₁-C₄, alkyle en C₄-C₂₂ interrompu par un oxygène éthérique, un azote ou un soufre, phényl- ou diphénylalkyle en C₇-C₂₂ éventuellement substitué par alkyle en C₁-C₄, halogène, alcoxy en C₁-C₄, méthylènedioxy, éthylènedioxy ou dialkylamino en C₁-C₄, phényle éventuellement substitué par alkyle en C₁-C₄ ou carboalcoxy en C₁-C₄, un radical de formule générale (IV) 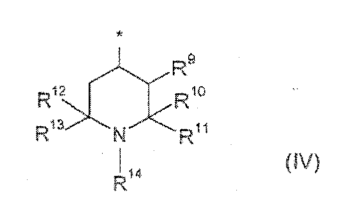 ou alkyle en C₁-C₂₂ contenant des radicaux hétérocycliques,
ou
- lorsque n = 2
R¹⁵ signifie alkylène en C₂-C₂₂, cycloalkylène en C₅- C₂₂, phénylalkylène en C₈-C₁₄, phénylène ou alkylène en C₄-C₃₀ interrompu par un oxygène éthérique, un azote, un soufre ou des hétérocycles à 5 ou 6 éléments,
et
(c) au moins un composé de formule générale (III), 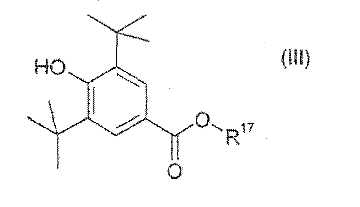 dans laquelle
R¹⁷ signifie alkyle en C₁-C₂₀, aryle,
et
(d) éventuellement des additifs supplémentaires,
les substituants R¹ à R¹⁷ pouvant à chaque fois être interrompus à une position quelconque par un ou plusieurs hétéroatomes, le nombre de ces hétéroatomes n'étant pas supérieur à 10, et/ou pouvant être substitués à chaque fois à une position quelconque, mais toutefois pas plus de cinq fois, par un alkyle en C₁-C₃₀, un alcoxy en C₁-C₂₀, un aryle, des hétérocycles, des hétéroatomes ou un halogène, ceux-ci pouvant également être substitués au plus deux fois, de préférence au plus une fois, avec les groupes mentionnés ou des aryles substitués.

2. Mélange selon la revendication 1, dans lequel des antioxydants sont également contenus en tant que composant (d).

3. Mélange selon la revendication 1 ou 2, avec R² correspondant à un mélange de groupes alkyle en C₁₄ à C₂₈, deux de ces groupes alkyle, qui ne peuvent différer de plus de deux atomes C, représentant à chaque fois au moins 30 % de ce mélange.

4. Mélange selon les revendications 1 à 3, dans lequel le composant (a) contient un composé oligomère comprenant une unité de répétition de formule générale (I) avec R³, R⁴, R⁵ et R⁶ = CH₃ et R² = alkyle en C₁₆-C₂₆.

5. Mélange selon les revendications 1 à 4, dans lequel le composant (b) contient un composé de formule générale (II), dans laquelle R³, R⁴, R⁵ et R⁶ représentent un méthyle et R² représente un atome d'hydrogène ou un alkyle en C₂-C₁₀.

6. Mélange selon les revendications 1 à 5, dans lequel le composant (c) contient un composé de formule générale (III), avec R¹⁷ = alkyle en C₁₆.

7. Mélange selon les revendications 1 à 6, dans lequel le composant (c) contient un composé de formule générale (III), avec R¹⁷ = 2,4-di-t-butyl-phényle.

8. Mélange selon les revendications 1 à 7, dans lequel le rapport en poids entre les composants (a) et/ou (b) et le composant (c) est compris entre 10:1 et 1:2.

9. Utilisation du mélange selon les revendications 1 à 8 pour la stabilisation de matériaux organiques non vivants contre l'effet de la lumière, de l'oxygène et/ou de la chaleur.

10. Utilisation selon la revendication 9 pour la stabilisation de plastiques, constitués d'au moins un polymère, choisi parmi les élastomères thermoplastiques, les polyoléfines, le polystyrène, les copolymères de styrène ou d'α-méthylstyrène, les polyesters, les polycarbonates, le polychlorure de vinyle, les polyacrylates, les polyméthacrylates, les polyuréthannes, ainsi que les mélanges physiques des polymères susmentionnés.

11. Utilisation selon la revendication 10 pour la stabilisation d'élastomères thermoplastiques à base d'oléfine.

12. Matériaux organiques non vivants, contenant au moins un mélange selon les revendications 1 à 8.

13. Articles, fabriqués à partir de matériaux organiques non vivants selon la revendication 12.

14. Procédé de stabilisation de matériaux organiques non vivants contre l'effet de la lumière, de l'oxygène et/ou de la chaleur, **caractérisé en ce qu'**au moins un mélange selon les revendications 1 à 8 est ajouté en une quantité efficace aux matériaux organiques non vivants.
